# EUROPEAN PATENT APPLICATION

(11) **EP 3 858 273 A1**
(43) Date of publication of application: **04.08.2021**
(21) Application number: 19864134.2
(22) Date of filing: 11.07.2019
(51) Int. Cl.: A61B 18/14, A61B 17/34

(54) **CAUTERY PUNCTURE NEEDLE**

(30) Priority: 28.09.2018 JP 2018183150
(71) Applicant: YOKOWO CO., LTD., Kita-ku Tokyo 114-8515 (JP)
(72) Inventor: SAKAI, Koichi, Tokyo 114-8515 (JP); YOSHIDA, Koki, Tokyo 114-8515 (JP)
(74) Representative: Gunzelmann, Rainer
(86) International application number: PCT/JP2019/027533
(87) International publication number: WO 2020/066226

(57) **Abstract**

The present invention provides a cautery puncture needle with which faults, such as damaging a peripheral tissue in a living body, are less likely to occur, puncturing a targeted narrow location in a membrane tissue, such as the interatrial septum, can easily be performed, and it can easily be confirmed that the distal end of the cautery puncture needle has reached an opposite side of the membrane tissue. The cautery puncture needle 10 includes a metal hollow pipe 15 having a hollow portion 2 communicating in the longitudinal direction and a distal end electrode portion 7 which is integrally installed at the distal end of the hollow pipe 15, which has an insertion distal end having a semi-ellipsoidal shape or the like, and through which a high-frequency current is to be passed, wherein a hole 9 communicating with the hollow portion 2 is formed at the side surface of the distal end electrode portion 7.

## Description

### Technical Field

The present invention relates to a medical cautery puncture needle to be used for cauterizing and puncturing a treatment part with a high-frequency wave (radio wave).

### Background Art

A catheter (puncture needle) having a sharp needle disposed at a distal end of the catheter is used as a medical treatment tool for puncturing a targeted treatment part in a living body. In recent years, a cautery puncture needle having an electrode that generates a high-frequency current disposed at the distal end of the catheter has been proposed (see Patent Literature 1). When a puncture needle having a needle disposed at the distal end of the catheter is used, a hole is opened at a targeted treatment part, such as a membrane part, with a needle by adding mechanical pushing force. On the other hand, when a cautery puncture needle is used, an electrode is brought into contact with a targeted treatment part, such as a membrane part, and a high-frequency current is passed through the electrode. Thereby, Joule heat is generated in a living body to cauterize the membrane part, and a hole can thereby be opened in the membrane part.

When a membrane part is punctured with a puncture needle which a mechanical needle is disposed, adjusting the force to be added is difficult in some cases, and therefore there is a need for sufficient consideration not to damage an unexpected location. On the other hand, when a membrane part is punctured with a cautery puncture needle, a part which is pressed with an electrode at the distal end is moved slightly toward the pressing direction, and a high-frequency current is thereafter passed through the electrode. Thereby, a hole is opened in the pressed part in the membrane part and the electrode at the distal end reaches the opposite side of the membrane part, and the part which has been pressed and moved slightly returns to the original position. Therefore, the use of a cautery puncture needle can reduce the possibility that faults, such as damaging an unexpected location, may occur even in an operation that requires extremely fine operation, such as, for example, puncturing the interatrial septum that isolates the right atrium and the left atrium.

When a minute membrane tissue, such as the interatrial septum, is punctured, there is a need to confirm that the distal end of a puncture needle has reached the opposite side of the membrane tissue. For example, a puncture needle for cauterization, wherein an injection hole for injecting a contrast medium is formed in a part which is on the base end side of a distal end electrode and is covered with an insulation layer, has been proposed (see Patent Literature 2).

### Citation List

### Patent Literature

Patent Literature 1: Japanese Patent Laid-Open No. 2008-237620
Patent Literature 2: National Publication of International Patent Application No. 2015-518752

### Summary of Invention

### Technical Problem

To confirm that the distal end electrode of the puncture needle proposed in Patent Literature 2 has reached the opposite side of a membrane tissue, there is a need to allow the injection hole to enter into the opposite side of the membrane tissue surely. However, allowing the injection hole to enter into the opposite side of the membrane tissue means that the distal end electrode enters into the opposite side of the membrane tissue more than necessary, and therefore a possibility that faults, such as damaging an unexpected location, occur increases. Moreover, when the distal end electrode is allowed to enter into the opposite side of the membrane tissue more than necessary, burdens on a living body are large.

The present invention has been completed in view of such circumstances, and an object of the present invention is to provide a cautery puncture needle with which faults, such as damaging a peripheral tissue in a living body, are less likely to occur, puncturing a targeted narrow location in a membrane tissue, such as the interatrial septum, can easily be performed, and it can easily be confirmed that the distal end of the cautery puncture needle has reached the opposite side of the membrane tissue.

### Solution to Problem

The first aspect of the present invention is a cautery puncture needle provided with: a metal hollow pipe having a hollow portion communicating in a longitudinal direction; and a distal end electrode portion through which a high-frequency current is to be passed, the distal end electrode portion integrally installed at a distal end of the hollow pipe, wherein a hole communicating with the hollow portion is formed at a side surface of the distal end electrode portion.

The second aspect of the present invention is the cautery puncture needle according to the first aspect, wherein an insertion distal end of the distal end electrode portion has a semi-ellipsoidal shape.

The third aspect of the present invention is the cautery puncture needle according to the second aspect, wherein the distal end electrode portion has an outer diameter of 0.4 to 0.9 mm.

The fourth aspect of the present invention is the cautery puncture needle according to any one of the first to the third aspects, wherein an opening of the hole has an oval shape or an elliptical shape extending in a longitudinal direction.

### Advantageous Effects of Invention

According to the above-described aspects of the present invention, a cautery puncture needle with which faults, such as damaging a peripheral tissue in a living body, are less likely to occur, puncturing a targeted narrow location in a membrane tissue, such as the interatrial septum, can easily be performed, and it can easily be confirmed that the distal end of the cautery puncture needle has reached the opposite side of the membrane tissue can be provided.

### Brief Description of Drawings

[Figure 1] Figure 1 is a partially sectional view schematically showing one embodiment of a cautery puncture needle of the present invention.
[Figure 2] Figure 2 is a partially perspective view schematically showing one embodiment of a cautery puncture needle of the present invention.
[Figure 3] Figure 3 is a partially perspective view schematically showing another embodiment of a cautery puncture needle of the present invention.
[Figure 4] Figure 4 is a partial side view schematically showing one embodiment of a cautery puncture needle of the present invention.
[Figure 5] Figure 5 is an overall perspective view schematically showing one embodiment of a cautery puncture needle of the present invention.
[Figure 6] Figure 6 is a partially sectional view showing one example of a state of injecting a liquid from a hole.
[Figure 7] Figure 7 is a partially sectional view showing another example of a state of injecting a liquid from a hole.
[Figure 8A] Figure 8A is a partially sectional view describing a procedure of preparing a distal end electrode portion.
[Figure 8B] Figure 8B is a partially sectional view describing a procedure of preparing a distal end electrode portion.
[Figure 8C] Figure 8C is a partially sectional view describing a procedure of preparing a distal end electrode portion.
[Figure 8D] Figure 8D is a partially sectional view describing a procedure of preparing a distal end electrode portion.

### Description of Embodiments

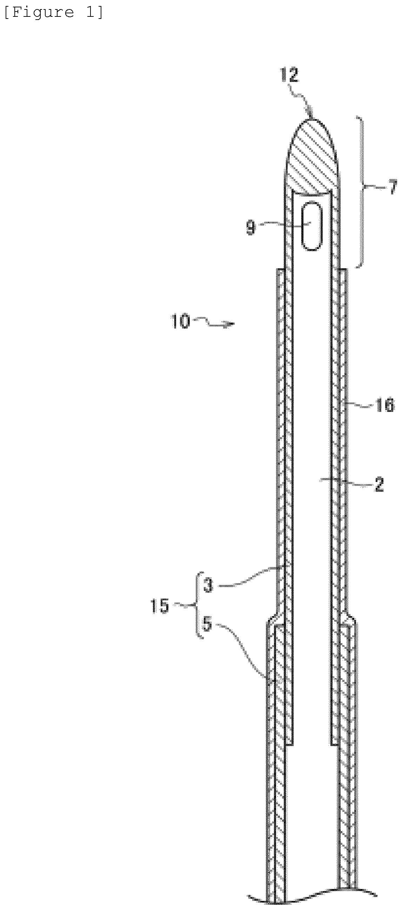

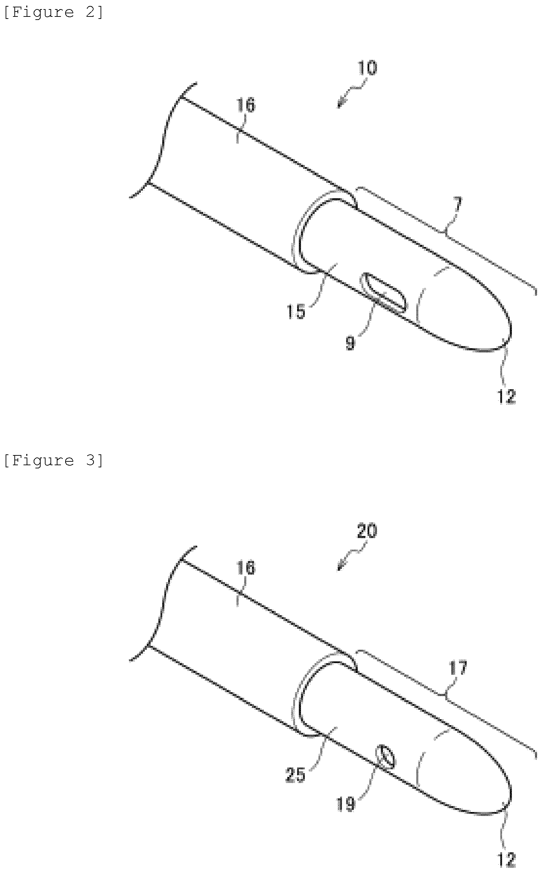

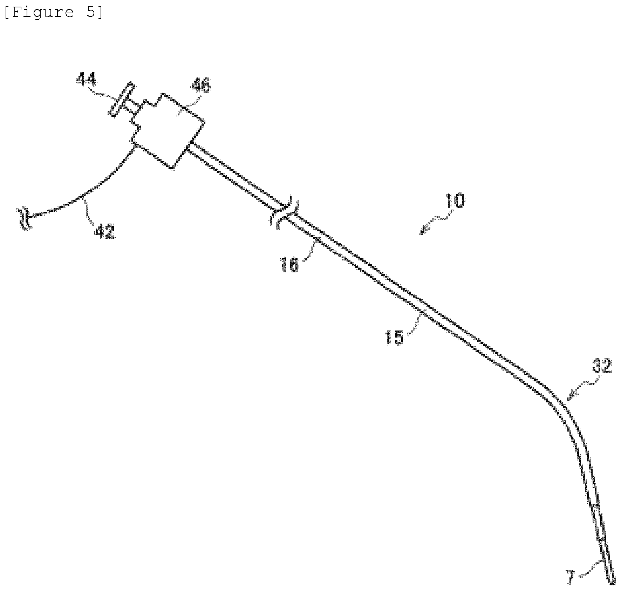

Hereinafter, embodiments of the present invention will be described, but the present invention is not limited to the following embodiments. Figure 1 is a partially sectional view schematically showing one embodiment of a cautery puncture needle of the present invention. Figure 2 is a partially perspective view schematically showing one embodiment of the cautery puncture needle of the present invention. As shown in Figure 1 and Figure 2, the cautery puncture needle 10 of the present embodiment includes a metal hollow pipe 15 having a hollow portion 2 communicating in the longitudinal direction and a distal end electrode portion 7. The distal end electrode portion 7 is integrally installed at the distal end of the hollow pipe 15 and is constituted in such a way that when a high-frequency current is passed through the distal end electrode portion 7, Joule heat is thereby generated in a living body. In addition, a hole 9 communicating with the hollow portion 2 in the hollow pipe 15 is formed at the side surface of the distal end electrode portion 7. The hollow portion 2 communicating with the hole 9 communicates to the operation base end side of the cautery puncture needle 10 in the longitudinal direction of the hollow pipe 15. Figure 5 is an overall perspective view schematically showing one embodiment of the cautery puncture needle of the present invention. As shown in Figure 5, a luer connector 46, for example, having a junction cable 42 and a syringe connecting portion 44, is installed on the operation base end side of the cautery puncture needle 10. The junction cable 42 is a connection means for electrically connecting a source of generating a high-frequency (radio wave (RF)) current, the hollow pipe 15 and the distal end electrode part 7. The syringe connecting portion 44 is a luer taper fitting portion to which a syringe is to be connected. When a syringe filled with a contrast medium is connected to the syringe connecting portion 44 to inject the contrast medium into the hollow portion, a liquid medicine can be injected outside from the hole 9 (Figure 1) formed at the side surface of the distal end electrode portion 7. That is, the hole formed at the side surface of the distal end electrode portion functions as an injection hole that injects a liquid medicine, such as a contrast medium, outside.

In the cautery puncture needle 10 of the present embodiment, the hole 9 that functions as an injection hole that injects a contrast medium is formed nearer to an insertion distal end 12 at the side surface of the distal end electrode portion 7 (Figure 1 and Figure 2). That is, according to the cautery puncture needle 10 of the present embodiment, a contrast medium can be injected from the hole 9 provided at a location nearer to the insertion distal end 12 to be inserted into a living body, and therefore it can easily be confirmed that the distal end electrode portion 7 has reached the opposite side of a membrane tissue, such as the interatrial septum.

The opening of the hole 9 formed at the side surface of the distal end electrode portion 7 of the cautery puncture needle 10 shown in Figure 1 and Figure 2 has an oval shape extending in the longitudinal direction of the hollow pipe 15. Figure 3 is a partially perspective view schematically showing another embodiment of the cautery puncture needle of the present invention. The cautery puncture needle 20 shown in Figure 3 includes a metal hollow pipe 25 and a distal end electrode portion 17. A hole 19 whose opening has a perfectly circular shape, the hole 19 communicating with a hollow portion in the hollow pipe 25, is formed at the side surface of the distal end electrode portion 17. As shown in Figure 1 and Figure 2, the opening of the hole 9 preferably has an oval shape or an elliptical shape extending in the longitudinal direction of the hollow pipe 15.

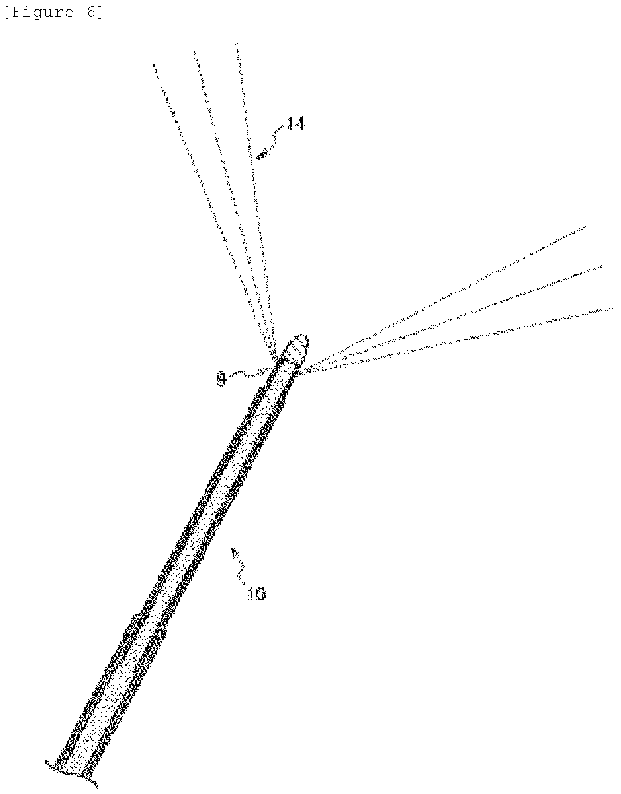

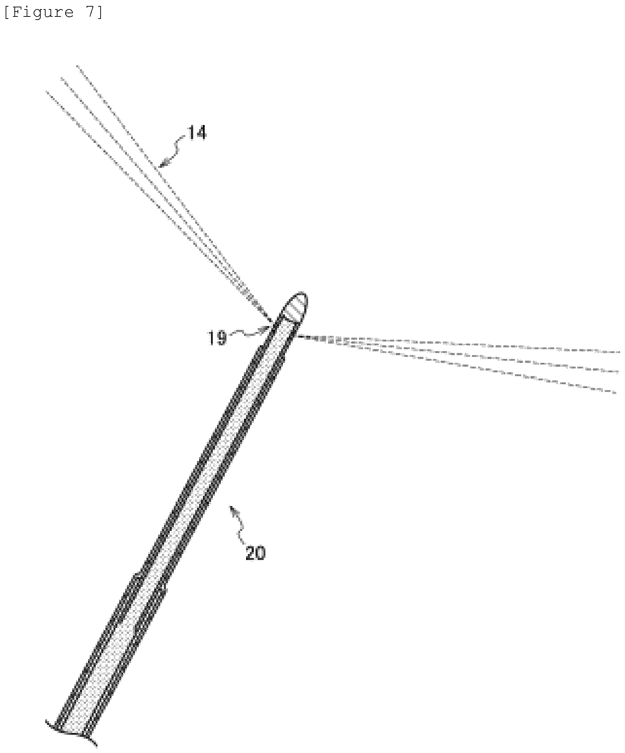

Figure 6 is a partially sectional view showing one example of a state of injecting a liquid from the hole. Figure 7 is a partially sectional view showing another example of the state of injecting a liquid from the hole. The opening of the hole 9 of the cautery puncture needle 10 shown in Figure 6 has an oval shape extending in the longitudinal direction of the hollow pipe (Figure 1 and Figure 2). On the other hand, the opening of the hole 19 of the cautery puncture needle 20 shown in Figure 7 has a perfectly circular shape (Figure 3). As shown in Figure 6 and Figure 7, the cautery puncture needle 10 (Figure 6) in which the opening of the hole 9 has an oval shape or an elliptical shape can inject a contrast medium 14 which is a liquid more forwarder in the insertion direction as compared to the cautery puncture needle 20 (Figure 7) in which the hole 19 has a perfectly circular shape. That is, when the opening of the hole is set in such a way as to have an oval shape or an elliptical shape, a contrast medium can be injected forwarder. Accordingly, even though the injection amount of a contrast medium is made smaller, an image can be photographed clearly by X-ray illumination. Thereby, it can be confirmed still more easily that the insertion distal end has reached the opposite side of a membrane tissue. In addition, the injection amount of a contrast medium can be reduced, and therefore burdens on a living body can also be reduced more.

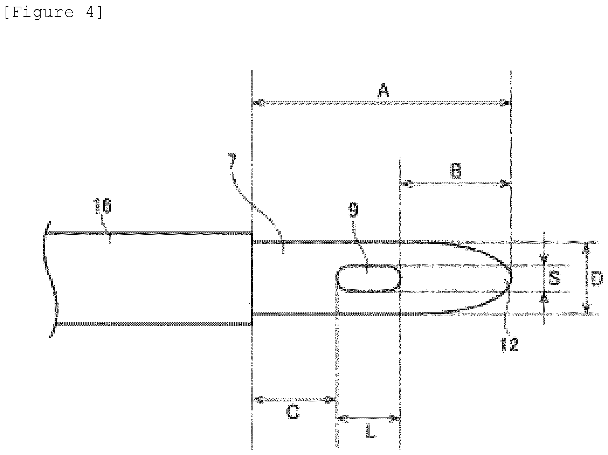

Figure 4 is a partial side view schematically showing one embodiment of the cautery puncture needle of the present invention. When the opening of the hole 9 has an oval shape or an elliptical shape, the ratio of the major axis diameter L to the minor axis diameter S (L/S ratio) of the hole 9 is preferably 1.06 to 6.00, and more preferably 1.19 to 3.67. By setting the L/S ratio to the above-described range, a contrast medium can be injected from the hole 9 forwarder in the insertion direction. The number of holes which are formed at the distal end electrode portion is not particularly limited, and is preferably set to 1 to 4, and more preferably 2 or 3. By setting the number of holes which are formed at the distal end electrode portion to 2 or 3, the strength of the distal end electrode portion can be retained more suitably.

As shown in Figure 1 and Figure 2, the insertion distal end of the distal end electrode portion 7 of the cautery puncture needle 10 preferably has a semi-ellipsoidal shape (artillery shell shape or semi-rugby-ball shape). When the insertion distal end has a semi-ellipsoidal shape that the outer diameter of the insertion distal end gradually reduces toward the insertion distal end 12, the insertion distal end 12 can be allowed to abut on a narrow location in a membrane tissue, such as the interatrial septum, more surely, and the contact area with the narrow location on which the insertion distal end 12 is allowed to abut can be made smaller. When the interatrial septum is supposed to be punctured, positional deviation of the insertion distal end 12 is liable to occur because the heart is in a state of beating, but when the insertion distal end has a semi-ellipsoidal shape, the pressing force of the insertion distal end 12 to the interatrial septum can be enhanced. Thereby, the positional deviation of the insertion distal end 12 is suppressed effectively, so that Joule heat can be concentrated on a desired narrow location in a membrane tissue, enabling opening a hole more surely.

The distal end electrode portion is formed with a metal because a high-frequency current is passed through it. The metal which forms the distal end electrode portion is not particularly limited as long as a high-frequency current can be passed through it, but is preferably a metal which can be photographed with high contrast by X-ray illumination so that a position in an operation part can easily be confirmed. Specific examples of the metal include platinum (Pt), iridium (Ir), and alloy of platinum and iridium (Pt-Ir alloy), and alloy of these metals and stainless steel (Pt-Ir-SUS alloy).

The hollow pipe 15 can be constituted by a distal end pipe 3 where the distal end electrode portion 7 is installed and a base end pipe 5 which is connected to the base end side of this distal end pipe 3 and has a slightly larger diameter than that of the distal end pipe (Figure 1). By constituting the insertion distal end side of the hollow pipe 15 by the distal end pipe 3 having a smaller diameter, a small hole that is a necessity minimum can be opened. By constituting the operation base end side of the hollow pipe 15 by the base end pipe 5 having a larger diameter, the movement at the operation part can be transmitted to the distal end more accurately.

The hollow pipe (distal end pipe, base end pipe) is formed with a metal having satisfactory flexibility. Examples of the metal include stainless steel, such as SUS302, SUS304V, and SUS316L, and various alloys, such as Nitinol, and Co-Cr. Among others, stainless steel, such as SUS304V, is preferable.

A covering layer 16 is formed on the surface of the hollow pipe 15 (Figure 1 and Figure 2). By forming the covering layer 16, unintended high-frequency wave (radio wave) cauterization to a blood vessel, an internal organ, or the like can be prevented, and sliding resistance of the cautery puncture needle 10 in a living body can be reduced, so that the operability can be improved. As the material for forming the covering layer 16, a hydrophobic resin material is preferable. Among others, a fluorine-based resin, such as PTFE, ETFE, or PFA, is preferable because the sliding resistance of the cautery puncture needle can be reduced more effectively by forming the covering layer with such a fluorine-based resin.

The size of each part of the distal end electrode portion 7 is preferably as follows (Figure 4). That is, the total length A of the distal end electrode portion 7 (length from insertion distal end 12 to covering layer 16) is preferably 1.16 to 2.50 mm. The length B from the insertion distal end 12 to the distal end of the opening of the hole 9 is preferably 0.50 to 1.10 mm. The length C from the base end of the opening of the hole 9 to the covering layer 16 is preferably 0.30 to 0.80 mm.

The outer diameter D of the distal end electrode portion 7 is preferably 0.4 to 0.9 mm, and more preferably 0.5 to 0.8 mm (Figure 4). When the interatrial septum is supposed to be punctured, injecting a contrast medium is not preferable in some cases for confirming that the distal end electrode portion including the insertion distal end of the cautery puncture needle has reached the opposite side (left atrium) of the interatrial septum. In such a case, when the outer diameter D of the distal end electrode portion 7 is in the above-described range, fluctuations in the right atrial pressure and in the left atrial pressure can be measured, so that whether the interatrial septum can be punctured or not can easily be decided. When the outer diameter D of the distal end electrode portion 7 is less than 0.4 mm, the decision is difficult even though the interatrial septum is punctured because fluctuations in the right atrial pressure and in the left atrial pressure are slight. On the other hand, when the outer diameter D of the distal end electrode portion 7 exceeds 0.9 mm, there is a tendency that the operability is lowered, and burdens on a living body are large.

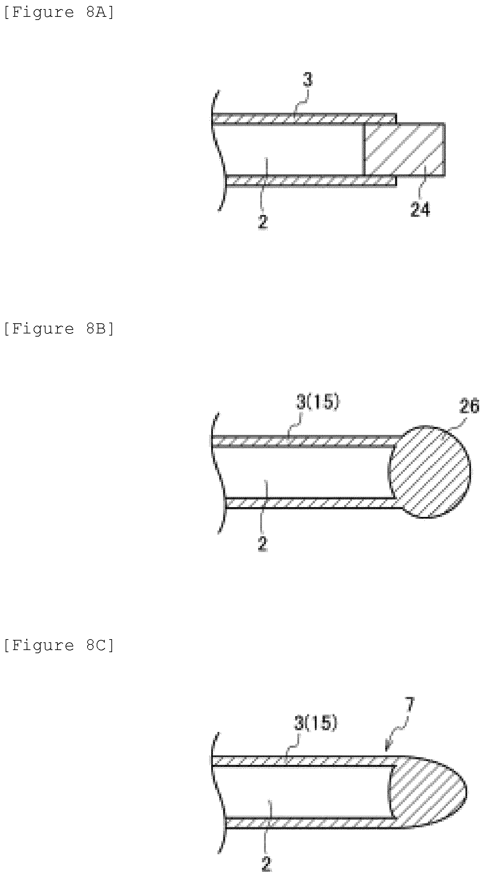

Next, a method for producing the cautery puncture needle of the present invention will be described. Figures 8A to 8D are partially sectional views describing a procedure of preparing the distal end electrode portion. To prepare the distal end electrode portion, as shown in Figure 8A, the distal end pipe 3 made of a metal, such as stainless steel, and having the hollow portion 2, and a columnar material 24 for an electrode are first prepared, and the material 24 for an electrode is inserted and fitted into one of the opening ends of the distal end pipe 3. The material 24 for an electrode is, for example, a member made of a metal, such as a Pt-Ir alloy. Subsequently, the fitted material 24 for an electrode and the opening end of the distal end pipe 3 are welded together by arc welding, such as plasma welding or Tig welding, to form a welded end portion 26 as shown in Figure 8B. The welded end portion 26 which is formed is an alloy of the metal constituting the distal end pipe 3 and the metal constituting the material 24 for an electrode (Figure 8A). The welded end portion 26 which is formed by the above-described welding is joined to the distal end pipe 3 (hollow pipe 15) substantially in a seamless manner.

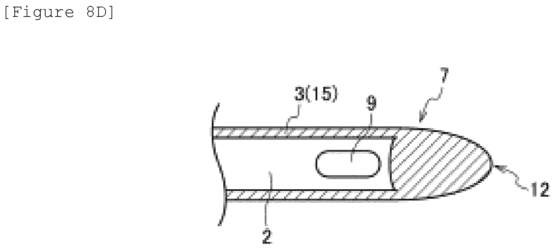

The distal end electrode portion 7 having a desired shape, such as a semi-ellipsoidal shape as shown in Figure 8C, can be formed by polishing the welded end portion 26 (Figure 8B) which has been formed. Subsequently, by opening the hole 9 communicating with the hollow portion 2 at the side surface of the distal end electrode portion 7 by laser processing or the like, the distal end electrode portion 7 as shown in Figure 8D can be formed. The distal end electrode portion 7 which is formed in this way is formed by joining the hollow pipe 15 and the insertion distal end 12 substantially in a seamless manner, and therefore faults, such as dropping of the insertion distal end 12 during use, are less likely to occur and safety is high. In addition, when the distal end electrode portion 7 is formed by joining the hollow pipe 15 and the insertion distal end 12 substantially in a seamless manner, the high-frequency current which has been passed is not stagnated, so that power loss can be reduced.

The base end of the distal end pipe having the distal end electrode portion prepared in the manner as described above is inserted into the opening end of the base end pipe having a slightly larger diameter than that of this distal end pipe to join these. Thereby, the hollow pipe having a hollow portion communicating from the insertion distal end to the operation base end side can be formed. The distal end pipe and the base end pipe are joined by, for example, laser welding, adhesion with an adhesive, a combination of these, or the like. If necessary, uneven processing is preferably performed on the outer peripheral surfaces of the distal end pipe and the base end pipe in advance because the covering layer which is disposed thereafter can be allowed to closely adhere in such a way as to be hard to deviate.

Subsequently, an adhesion binder is applied on the outer peripheral surface of the hollow pipe, and the hollow pipe is inserted into a heat-shrinkable tube made of a fluororesin, such as PTFE. When the heat-shrinkable tube is shrunken by moderate heating, the covering layer can thereby be formed at a predetermined location on the outer peripheral surface of the hollow pipe in such a way as to closely adhere. Thereafter, as shown in Figure 5, a desired location on the hollow pipe 15 is bent to form a bending portion 32, and the operation part, such as the luer connector 46, is connected to the operation base end of the hollow pipe 15, and the cautery puncture needle 10 of the present embodiment can thereby be obtained.

### Industrial Applicability

The cautery puncture needle of the present invention is useful as, for example, a puncture needle for puncturing the interatrial septum that isolates the right atrium and the left atrium.

### Reference Signs List

2: Hollow portion
3: Distal end pipe
5: Base end pipe
7, 17: Distal end electrode portion
9, 19: Hole
10, 20: Cautery puncture needle
12: Insertion distal end
14: Contrast medium
15, 25: Hollow portion
16: Covering layer
24: Material for electrode
26: Welded end part
32: Bending portion
42: Junction cable
44: Syringe connecting portion
46: Luer connector

## Claims

1. A cautery puncture needle comprising:
a metal hollow pipe having a hollow portion communicating in a longitudinal direction; and
a distal end electrode portion through which a high-frequency current is to be passed, the distal end electrode portion integrally installed at a distal end of the hollow pipe, wherein
a hole communicating with the hollow portion is formed at a side surface of the distal end electrode portion.

2. The cautery puncture needle according to claim 1, wherein an insertion distal end of the distal end electrode portion has a semi-ellipsoidal shape.

3. The cautery puncture needle according to claim 2, wherein the distal end electrode portion has an outer diameter of 0.4 to 0.9 mm.

4. The cautery puncture needle according to any one of claims 1 to 3, wherein an opening of the hole has an oval shape or an elliptical shape extending in a longitudinal direction.
